# EUROPÄISCHE PATENTANMELDUNG

(11) **EP 1 022 033 A1**
(43) Veröffentlichungstag der Anmeldung: **26.07.2000**
(21) Anmeldenummer: 99890350.4
(22) Anmeldetag: 02.11.1999
(51) Int. Cl.: A61M 25/10, A61B 17/12

(54) **Katheter zur intravaskulären Verbindung zweier Gefässabschnitte**

(30) Priorität: 02.11.1998 AT 181798
(71) Anmelder: Pro-Med Technology Consult medizinisch-technische Geräte GmbH, 2340 Mödling (AT)
(72) Erfinder: Mohl, Werner, Prof. Dr., 2571 Altenmarkt/Tennenberg (AT); Claassen, Dirk Peter, Dipl.-Ing., 8045 Graz (AT)
(74) Vertreter: Laminger, Norbert, Mag.

(57) **Zusammenfassung**

Ein Katheter zur intravasculären Verbindung zweier Gefäßabschnitte ist mit zwei voneinander beabstandeten, ausweitbaren Abdichtelementen auf einer durchströmbaren Verbindungsleitung (3) und einer Leitung (5) zur Fluidzufuhr zu den Abdichtelementen versehen. Um bei größtmöglicher Einfachheit der Handhabung, insbesondere beim Einsetzen und Entfernen, und Anpaßbarkeit an das jeweilige Gefäß die Operationsstelle optimal von Blut freizuhalten, ist zumindest eines der Abdichtelemente (4, 9) mit zumindest einer Öffnung (8, 10) zum unidirektionalen Fluidaustritt in Richtung auf den Bereich zwischen den beiden Abdichtelementen versehen.

## Beschreibung

Die Erfindung betrifft einen Katheter zur intravasculären Verbindung zweier Gefäßabschnitte, mit zwei voneinander beabstandeten, ausweitbaren Abdichtelementen auf einer durchströmbaren Verbindungsleitung und einer Leitung zur Fluidzufuhr zu den Abdichtelementen.

Bei der Herstellung von Anastomosen, beispielsweise an Herzkranzgefäßen oder auch an periphären Gefäßen, sowie der Verbindung zweier Gefäßabschnitte ist zur Freihaltung des Operationsgebietes von Blut die Abdichtung der jeweiligen Gefäßabschnitte notwendig. Um diese Maßnahme möglichst ohne Klemmung und äußeren Druck auf das Gefäß zu setzen, ist ein Katheter entwickelt worden, der eine flexible, durchströmbare Leitung aufweist, an deren Enden runde Dichtelemente aus Silikon vorgesehen sind. Dieser Katheter wird in das Gefäß eingeführt und die Silikon-Dichtelemente dichten das Gefäß vor und hinter der Operationsstelle ab, während Blut weiterhin durch die Leitung hindurch strömen kann und die Bereiche stromabwärts der Operationsstelle versorgt werden. In der EP-A-0 839 550 ist ein Bypass-Katheter offenbart, bei dem die Abdichtelemente durch mittels eines Gases aufblasbare Ballons gebildet sind, was das Einsetzen und Entfernen des Katheters erleichtert und auch die individuelle Anpassung an den Gefäßdurchmesser erlaubt.

Die Aufgabe der vorliegenden Erfindung war ein Katheter, mit dem bei größtmöglicher Einfachheit der Handhabung, insbesondere beim Einsetzen und Entfernen, und Anpaßbarkeit an das jeweilige Gefäß die Operationsstelle optimal von Blut freigehalten werden kann und trotzdem ein Perfusion des Gefäßes, vorzugsweise mit Blut, erlaubt.

Zur Lösung dieser Aufgabe ist der eingangs beschriebene Katheter erfindungsgemäß dadurch gekennzeichnet, daß zumindest eines der Abdichtelemente mit zumindest einer Öffnung zum unidirektionalen Fluidaustritt in Richtung auf den Bereich zwischen den beiden Abdichtelementen versehen ist. Damit ist das Einsetzen und Entfernen bei geringstmöglichen Abmessungen möglich, die Dichtwirkung ist durch die steuerbare, durch die Fluidzufuhr hervorgerufene Ausweitung gewährleistet und durch die Rückführung des Fluides, vorzugsweise eines Gases wie etwa Kohlendioxid, auf die Operationsstelle hin wird diese von Blut freigehalten. Dabei ist der Fluidverbrauch durch die doppelte Ausnutzung und sehr gezielte und begrenzte Zufuhr des Fluides relativ gering. Weiters wird, speziell bei Gaszufuhr, die Anastomose durch den Gasstrahl nicht deformiert.

Gemäß einer ersten Ausführungsform der Erfindung ist das oder jedes Abdichtelement als vorzugsweise elastische, ausweitbare Manschette um das Katheterende ausgeführt, welche Manschette in Richtung auf den Bereich zwischen den beiden Abdichtelementen hin offen ist. Dies ergibt eine in der Herstellung sehr einfache und mit geringstem Durchmesser anzufertigende Konstruktion mit dennoch sicherer Abdichtung des Operationsgebietes. Durch die dem Operationsgebiet zugekehrte Öffnung ist der größtmöglich Fluidstrom in dieses Gebiet zum Verdrängen von Blut gewährleistet.

Vorteilhafterweise ist dabei vorgesehen, daß die Leitung zur Fluidzufuhr zumindest eine Fluidaustrittsöffnung im von der Manschette überdeckten Bereich aufweist. Damit ist schon zu Beginn der Fluidzufuhr bzw. mit geringsten Fluidmengen die optimale Abdichtwirkung und Rückströmung ins Operationsgebiet sichergestellt.

Eine andere Ausführungsform ist dadurch gekennzeichnet, daß das oder jedes Abdichtelement aus einem vorzugsweise elastischen, ausweitbaren Ballon um das Katheterende besteht, welcher Ballon zumindest eine auf den Bereich zwischen den beiden Abdichtelementen hin gerichtete Fluidaustrittsöffnung aufweist. Bei dieser Ausführungsform ist auch bei geringen Fluidmengen eine rasche Abdichtung des Operationsbereiches zu erzielen, da das gesamte zugeführte Fluid zur Aufweitung des Abdichtelementes beiträgt und nichts ungenutzt abströmt.

Die bestmögliche Freihaltung des Operationsgebietes von Blut ist bei dieser Variante möglich, wenn der oder jeder Ballon mit einer über den Umfang verteilten Gruppe von Fluidaustrittsöffnungen versehen ist.

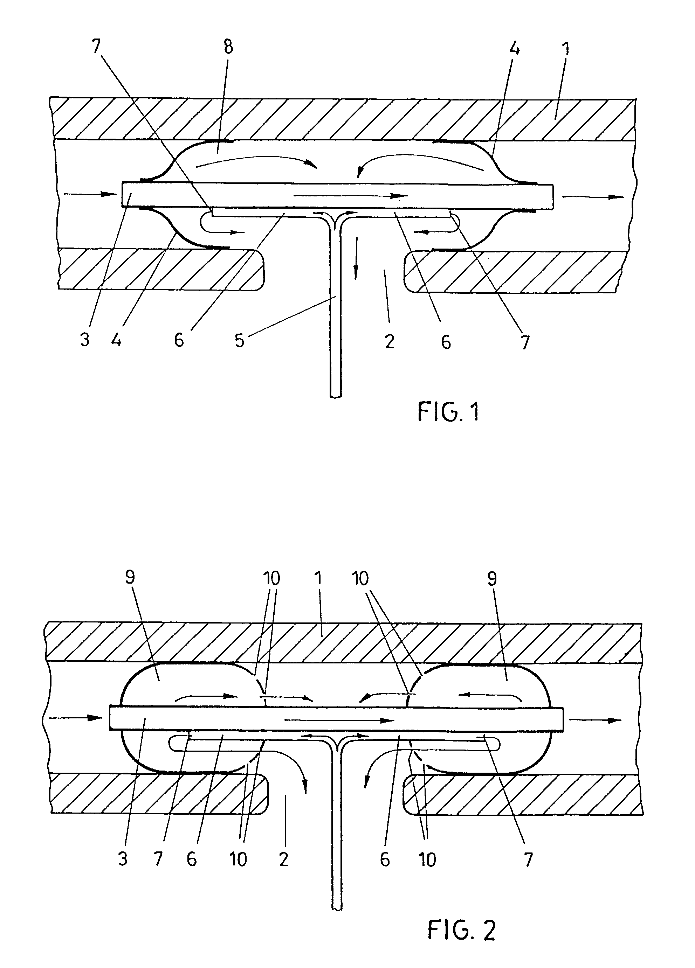

In der nachfolgenden Beschreibung soll die Erfindung beispielhaft anhand der beigefügten Zeichnungen erläutert werden. Dabei zeigt die Fig. 1 eine erste Ausführungsform des Katheters mit ausweitbarer Manschette und Fig. 2 ist eine weitere Ausführungsform mit aufblasbaren Ballons.

Die Fig. 1 zeigt ein Blutgefäß 1, beispielsweise ein Herzkranzgefäß oder periphäres Gefäß, im Querschnitt. Durch einen Einschnitt 2 wurde der erfindungsgemäße Katheter eingesetzt, der in der Ausführungsform der Fig. 1 eine vom Blut durchströmbaren Verbindungsleitung 3 aufweist, welche die Gefäßabschnitte vor und hinter dem Einschnitt 2 verbindet und somit die Blutversorgung des stromab des Einschnittes gelegenen Gebietes sicherstellt. Der Katheter könnte auch in die einander zugewandten Enden zweier operativ zu verbindender Gefäße eingesetzt werden, wobei dann über die Verbindungsleitung 2 die Blutverbindung hergestellt ist.

An beiden Enden der Verbindungsleitung 2 ist als ausweitbares Abdichtelement je eine, zum gegenüberliegenden Ende der Verbindungsleitung 3 hin offe Manschette 4 vorgesehen, die sich unter dem Druck eines durch die Leitung 5 zugeführten Fluides, vorzugsweise eines Gases, insbesondere Kohlendioxid, aufbläht und an die Innenwand des Gefäßes 1 dichtend anlegt. Dazu sind zwei Leitungsabschnitte 6 bis in die Bereiche des Katheters geführt, die von der Manschette 4, zumindest in deren anliegender Stellung, überdeckt werden und diese Leitungsabschnitte 6 weisen in diesem Bereich zumindest eine Fluidaustrittsöffnung 7 auf. Die Manschette 4 selbst ist aus einem elastischen Material angefertigt und/oder im Ruhezustand um die Verbindungsleitung 3 zusammengefaltet.

Das unter die Manschette 4 eingeblasene Fluid strömt, nachdem es die Manschette 4 ausgeweitet und an die Gefäßinnenwand gedrückt hat, durch den Ringspalt 8 zwischen dem Außenrand der Manschette 4 und der Verbindungsleitung 3 in den Bereich zwischen den Manschetten 4 hin, d.h. in Richtung auf den Bereich des Einschnittes 2 hin, und hält so diesen Bereich frei von Blut.

Bei der Ausführungsform der Fig. 2 sind anstelle der Manschette 4 an den Enden der Verbindungsleitung 3 aufblasbare Ballons 9 vorgesehen. Die parallel zur Verbindungsleitung 3 verlaufenden Leitungsabschnitte 6 führen dabei bis in das Innere dieser Ballons 9. Dadurch wird die gesamte Fluidmenge optimal für die Abdichtung der Operationsstelle verwendet. Um auch hier die Operationsstelle blutfrei zu halten, sind vorzugsweise mehrere und vorzugsweise um den Umfang der Verbindungsleitung 3 verteilte Fluidaustrittsöffnungen 10 in Richtung auf den Bereich zwischen den Ballons 9 hin vorgesehen.

Bei beiden Ausführungsformen ist selbstverständlich zum Funktionieren der Abdichtung notwendig, daß der Strömungswiderstand der Fluidaustrittsöffnungen 8 bzw. 10 der Manschetten 4 bzw. der Ballons 9 größer ist als der Strömungswiderstand der Leitung 5 und der Leitungsabschnitte 6.

## Patentansprüche

1. Katheter zur intravasculären Verbindung zweier Gefäßabschnitte, mit zwei voneinander beabstandeten, ausweitbaren Abdichtelementen auf einer durchströmbaren Verbindungsleitung und einer Leitung zur Fluidzufuhr zu den Abdichtelementen, dadurch gekennzeichnet, daß zumindest eines der Abdichtelemente (4, 9) mit zumindest einer Öffnung (8, 10) zum unidirektionalen Fluidaustritt in Richtung auf den Bereich zwischen den beiden Abdichtelementen versehen ist.

2. Katheter nach Anspruch 1, dadurch gekennzeichnet, daß das oder jedes Abdichtelement als vorzugsweise elastische, ausweitbare Manschette (4) um das Katheterende ausgeführt ist, welche Manschette in Richtung auf den Bereich zwischen den beiden Abdichtelementen hin offen ist.

3. Katheter nach Anspruch 2, dadurch gekennzeichnet, daß die Leitung (5) zur Fluidzufuhr zumindest eine Fluidaustrittsöffnung (7) im von der Manschette (4) überdeckten Bereich aufweist.

4. Katheter nach Anspruch 1, dadurch gekennzeichnet, daß das oder jedes Abdichtelement aus einem vorzugsweise elastischen, ausweitbaren Ballon (9) um das Katheterende besteht, welcher Ballon zumindest eine auf den Bereich zwischen den beiden Abdichtelementen hin gerichtete Fluidaustrittsöffnung (10) aufweist.

5. Katheter nach Anspruch 4, dadurch gekennzeichnet, daß der oder jeder Ballon (9) mit einer über den Umfang verteilten Gruppe von Fluidaustrittsöffnungen (10) versehen ist.
